# EUROPEAN PATENT APPLICATION

(11) **EP 4 599 777 A1**
(43) Date of publication of application: **13.08.2025**
(21) Application number: 25155255.0
(22) Date of filing: 31.01.2025
(51) Int. Cl.: A61B 17/3209, A61F 2/04, A61F 2/94, A61B 17/00, A61B 18/00

(54) **PROSTATIC URETHRA IMPLANT FOR TREATING PROSTATE ENLARGEMENT**

(30) Priority: 12.02.2024 US 202463552207 P
(71) Applicant: Medi-Tate Ltd., 3850169 Hadera (IL)
(72) Inventor: KILEMNIK, Ido, 4286500 Haniel (IL)
(74) Representative: Hoener, Matthias

(57) **Abstract**

The disclosed technique relates to systems and methods for relieving a prostate enlargement (e.g., as a result of benign prostatic hyperplasia), in general, and to systems and methods for enlarging the prostatic urethra and for anchoring the median lobe of the prostate.

## Description

### FIELD OF THE DISCLOSED TECHNIQUE

The disclosed technique relates to systems and methods for relieving a prostate enlargement (e.g., as a result of benign prostatic hyperplasia), in general, and to systems and methods for enlarging the prostatic urethra and for anchoring the median lobe of the prostate.

### BACKGROUND OF THE DISCLOSED TECHNIQUE

The prostate is a walnut-sized gland that forms part of the male reproductive system. The prostate is located in front of the rectum and just below the bladder, where urine is stored. The urethra is the canal connecting the bladder to the penis through which urine can be passed out of the body. In the male reproductive system, the ejaculatory ducts are the tubes connecting the testes to the urethra through which semen can also be passed out of the body. The prostate surrounds a portion of the urethra (herein referred to as the prostatic urethra), with the ejaculatory ducts also passing through the prostate and connecting with the prostatic urethra. The prostate itself is anatomically composed of four lobes, the anterior lobe, the median lobe, the posterior lobe and the lateral lobes. The anterior, median and posterior lobes surround the urethra whereas the ejaculatory ducts pass through the median and posterior lobes. The lateral lobes are positioned around the anterior, median posterior lobes. An anatomical landmark on the prostatic urethra used in the classification of certain urethral development disorders is known as the verumontanum of the seminal colliculus. The verumontanum is located between the ejaculatory ducts, the prostatic utricle and the prostatic ducts, being inferior to the urethral crest.

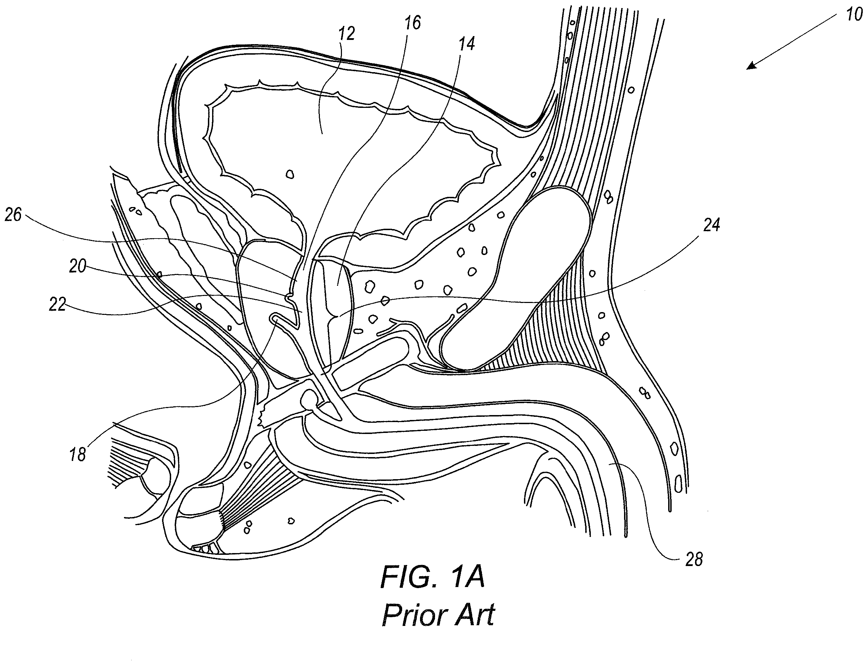

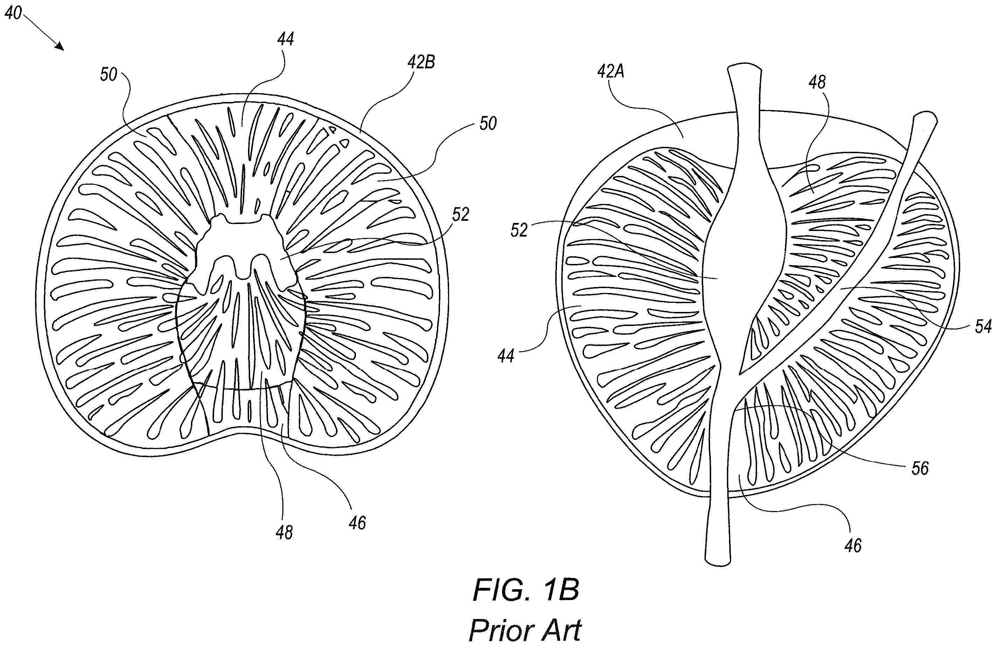

Reference is now made to Figures 1A and 1B, which are schematic illustrations respectively of the male reproductive system and the prostate, generally referenced 10 and 40 respectively, as is known in the prior art. With reference to Figure 1A, shown is a schematic illustration of the male reproductive system from a sagittal view. As can be seen, a urinary bladder 12 connects with a urethra 16 which flows to a penis 28. A prostate 14, inferior to urinary bladder 12, surrounds urethra 16. The portion of urethra 16 which is surrounded by prostate 14 is shown as a prostatic urethra 24. Plurality of ejaculatory ducts 18 couple with urethra 16 in prostatic urethra 24. Shown within the section of prostatic urethra is a urethral crest 26, a prostatic utricle 20 and a verumontanum 22. With reference to Figure 1B, shown is a schematic illustration of the prostate from a sagittal view 42A and a transverse view 42B. Identical elements in sagittal view 42A and transverse view 42B are shown using the same reference numbers. As can be seen from sagittal view 42A, an anterior lobe 44, a posterior lobe 46 and a median lobe 48 surround a urethra 52. An ejaculatory duct 54 passes through posterior lobe 46 and median lobe 48. Shown as well is a verumontanum 56 on the proximal end of posterior lobe 46. As can be seen from transverse view 42B, plurality of lateral lobes 50 surround anterior lobe 44, posterior lobe 46 and median lobe 48, with urethra 52 flowing between the various lobes of the prostate.

Common medical conditions of the prostate can include inflammation, non-cancerous enlargement of the prostate and prostate cancer. Non-cancerous enlargement of the prostate, also known as prostate enlargement occurs primarily in older men (in general +50) and is a medical condition in which the prostate grows in size but not due to metastasis or uncontrollable cell reproduction. As the prostate enlarges and grows in size, as can be seen from its anatomical position in human males such as shown in Figure 1A, it can apply pressure on the urethra, in particular the prostatic urethra, as well as adjacent anatomical sites such as the bladder neck (the inferior part of the bladder which connects with the urethra) and the ejaculatory ducts. In general, when the prostate enlarges, it is the lateral lobes which enlarge and place pressure on the prostatic urethra. However in some cases, the median lobe may also enlarge as well. Prostate enlargement can lead to a number of medical problems such as Benign Prostatic Hyperplasia (herein abbreviated BPH), prostatic Bladder Neck Obstruction (herein abbreviated BNO) and the like. BPH cause increased pressure on the prostatic urethra, making it difficult and painful to urinate. BNO can cause a complete obstruction of the prostatic urethra and an inability for the muscles around the bladder neck (for example the internal urethral sphincter) to relax and contract, making it nearly impossible to urinate and usually requiring medical intervention to relieve the bladder of urine.

Treatments for medical problems resulting from prostate enlargement range from medications taken orally (to reduce the size of the prostate by reducing hormone production), various kinds of stents and implants to enlarge the prostatic urethra, the use of catheter to enable a path from the bladder to the penis to expel urine as well as surgical procedures for removing either a portion of the prostate (such as transurethral resection of the prostate) or the entire prostate (such as a prostatectomy). Stents and implants for opening up the prostatic urethra are known in the art. PCT Patent Application Publication No. WO 2006/040767 A1 to Kilemnik and entitled "Prostate Treatment Stent" is directed to a tissue dissecting implant. The implant is spring-shaped and includes a plurality of rings elastically coupled there-between. Adjacent rings apply pressure on tissues caught between the rings, thereby pinching the caught tissues and inducing necrosis.

US Patent Application Publication No. 2011/0276081 A1 to Kilemnik and entitled "Radial Cutter Implant" is directed to an implant for applying radial forces on the tissues surrounding it. The implant includes wires for applying radial pressure on the surrounding tissues. Each of the wires extends in a different radial direction, and therefore, each wire applies pressure on different tissues. The implant can further include a longitudinal central tube, such that the wires are coupled with a proximal end and a distal end of the tube. The tube supports the wires and provides structural stability to the implant. The distal end of the wires is positioned within the bladder of the subject, and may irritate the bladder.

US Patent No. 11,304,724 B2 to Kilemnik and entitled "Incising Implant For The Prostatic Urethra" is directed to an implant for creating incisions in the prostatic urethra of a subject. The implant includes at least two closed-shaped wires, each of which has a proximal section, a distal section and two longitudinal sections extending between the proximal section and the distal section. Each of the closed-shaped wires is elastic and thus compressible into a compressed configuration. Each of the longitudinal sections of each of the wires is adjoined with another longitudinal section of another one of the wires. In an open configuration, the implant incises tissue in the prostatic urethra, with the wires diverging at the distal sections such that the distal sections form a simple closed curve shape at a terminus of the implant.

US Patent No. 8,715,239 B2 to Lamson et al., entitled "Devices, Systems, and Methods for Treating Benign Prostatic Hyperplasia and Other Conditions" is directed to a system including a rigid introducer device that is useable to facilitate insertion of an implant into the prostate gland. The implant includes a proximal anchor connected to a distal anchor by a tensioning element and the introducer device includes a rigid elongate body that is insertable into the subject's urethra. The introducer device also includes a rigid scope lumen configured to receive a cystoscope or other endoscopic device and a rigid working lumen. The working lumen is used to place a prostate compression implant and has an outlet through which the implant can be advanced through the wall of the urethra and to a position within or near the prostate gland.

The prior art provides solutions to the pressure exerted on the prostatic urethra when the lateral lobes of the prostate enlarge, which is the more common form of BPH. However when the lateral lobes as well as the median lobe of the prostate enlarge, further complications may arise. As described below, an enlarged median lobe may exhibit sufficient movement within the prostatic urethra to obstruct the bladder neck after urination, thereby causing BNO. Regarding regular urination in males, as the muscles around the bladder contract and squeeze the bladder, the muscles around the bladder neck (such as the internal urethral sphincter) relax to let urine into the prostatic urethra. As urine is expelled from the bladder, air pressure is generated in the bladder which forces urine out of the bladder into the urethra. Once urination is finished and the muscles around the bladder relax and the muscles around the bladder neck contract to close the bladder neck, negative air pressure and suction may be generated in the prostatic urethra. In the case of BPH when only the lateral lobes of the prostate enlarge, the negative air pressure may have no effect on the opening of the bladder neck. However in the case of BPH when the lateral and median lobes enlarge, the negative air pressure (i.e., suction) may pull the enlarged median lobe towards the bladder neck, thereby causing BNO as well. Prior are treatment methods in such cases (BPH and BNO with an enlarged median lobe) usually use some form of laparoscopic surgery for removing a portion of the median lobe to ease symptoms. What is needed therefore is a minimally invasive treatment, such as an implant, capable of opening up the prostatic urethra to treat the symptoms of BPH while also preventing the median lobe from blocking the bladder, thereby also treating the symptoms of BNO.

### SUMMARY OF THE DISCLOSED TECHNIQUE

It is an object of the disclosed technique to provide methods and systems for a prostatic urethra implant for treating prostate enlargement when both the lateral lobes and the median lobe enlarge.

### BRIEF DESCRIPTION OF THE DRAWINGS

The disclosed technique will be understood and appreciated more fully from the following detailed description taken in conjunction with the drawings in which:
Figures 1A and 1B are schematic illustrations of the male reproductive system and the prostate, as is known in the prior art;
Figures 2A, 2B and 2C are schematic illustrations of a prostatic urethra implant for creating incisions in the tissues of the inner wall of the prostatic urethra and for compressing the median lobe, constructed and operative in accordance with an embodiment of the disclosed technique;
Figures 3A, 3B and 3C are schematic illustrations of a second prostatic urethra implant for creating incisions in the tissues of the inner wall of the prostatic urethra, constructed and operative in accordance with another embodiment of the disclosed technique;
Figure 4 is a schematic illustration of a third prostatic urethra implant for creating incisions in the tissues of the inner wall of the prostatic urethra, constructed and operative in accordance with a further embodiment of the disclosed technique;
Figures 5A and 5B are schematic illustrations of a proximal niche of a proximal cap of a prostatic urethra implant, constructed and operative in accordance with yet another embodiment of the disclosed technique;
Figures 6A and 6B are schematic illustrations of a fourth prostatic urethra implant for creating incisions in the tissues of the inner wall of the prostatic urethra, constructed and operative in accordance with yet a further embodiment of the disclosed technique;
Figure 7 is a schematic illustration of a fifth prostatic urethra implant for creating incisions in the tissues of the inner wall of the prostatic urethra, constructed and operative in accordance with yet another embodiment of the disclosed technique;
Figure 8 is a schematic illustration showing the placement of a prostatic urethra implant within the prostatic urethra, constructed and operative in accordance with yet a further embodiment of the disclosed technique; and
Figures 9A-9L are schematic illustrations of a method for deploying and for extracting a prostatic urethra implant, operative in accordance with yet another embodiment of the disclosed technique.

### DETAILED DESCRIPTION OF THE EMBODIMENTS

The disclosed technique overcomes the disadvantages of the prior art by providing a prostatic urethra implant positioned within the prostatic urethra (or in the vicinity thereof, for example, in the bladder neck). The implant includes wires which apply a radial force on the surrounding tissues of the inner wall of the prostatic urethra. Over time, the wires cause longitudinal incisions in the prostatic urethra and induce infraction (i.e., tissue death due to lack of blood supply) therein. The incisions and resulting infarction relieve constriction of the prostatic urethra by killing a portion of the tissues in the prostatic urethra causing blockage of the urethra. The implant also includes at least one anchor for simultaneously holding the implant in place and preventing its migration into the bladder while also applying pressure to the median lobe of the prostate and thus preventing it from moving (due to suction) after urination. The implant thus provides a solution to a constricted prostatic urethra due to BPH as well as to preventing BNO due to median lobe enlargement.

As described above, in some cases of BPH, not only do the lateral lobes of the prostate enlarge in prostate enlargement but the median lobe as well can enlarge. In such cases, the proximate side of the median lobe closest to the prostatic urethra can form a bulbous and dangling end. Due to the suction created when urinating, when a male ceases to urinate, the suction from the bladder may pull the bulbous and dangling end towards the bladder neck, thereby causing BNO. Thus the bulbous and dangling end of the median has sufficient movement to block the bladder neck even in cases where the prostatic urethra has been relieved of the general constriction caused by enlargement of the lateral lobes of the prostate.

In accordance with an embodiment of the disclosed technique, the implant is formed by three (or more) closed-shape incising wires having at least one additional closed-shape anchoring wire acting as an anchor for restricting movement of the bulbous and dangling end of an enlarged median lobe as well as preventing the implant from migrating towards the bladder. The incising wires and the anchoring wire are all coupled with a hollow cap, providing a base structure of the implant while also enabling urine to pass through the implant. The shape of each incising wire can be broadly divided into a proximal section, a distal end section and two lateral sections extending between the proximal and distal sections. The shape of the anchoring wire resembles the outline of a leaf or teardrop having a distal curvature for applying pressure radially outward. Each wire (incising and anchoring) is made from an elastic and shape memory material allowing it to be compressed into a sheath and then expanding to its original shape when released from the sheath.

The lateral sections of each incising wire are adjoined with the lateral sections of adjacent incising wires. Thus, the incising wires are coupled to each other to form a wire frame. Each incising wire forms a face of the wire frame with the adjoined lateral sections forming the edges of the wire frame. The edges of the wire frame apply outward radial pressure on the tissues of the inner wall of the prostatic urethra, thereby creating longitudinal incisions that relieve urethral constriction and increase the urinal passage.

The incising wires, when applying pressure on the surrounding tissues, are pressed against one another (i.e., each wire is pressed against the adjacent wires to which it is adjoined to at the respective lateral sections). Thus the wire frame is self-supporting with the incising wires supporting each other. When an incising wire applies a force on a tissue, the tissue applies an opposite force having the same magnitude (in accordance with Newton's third law of motion). The incising wire is thus pressed against the adjoined adjacent incising wires. These adjoined incising wires, in turn, are pushed against the inner wall of tissues in the prostatic urethra. In this manner, the wire frame is self-supporting, obviating the need for an additional supporting element, such as a central support tube. Additionally, each edge of the wire frame is formed by two adjoined incising wires, thus doubling the pressure applied to the tissues and allowing for thinner wires.

In a first embodiment of the disclosed technique, the implant has two anchoring wires, each anchoring wire having a shape resembling the outline of a leaf, a leaflet or a teardrop. A first anchoring wire is used to anchor the wire frame of the incising wires such that the implant does not migrate to the bladder. A second anchoring wire is used to anchor the bulbous and dangling end of the median lobe such that it does not move when suction is generated after urination. The shape of both the first and second anchoring wires has a curvature which curves outwards, thereby being able to apply an outward radial pressure. The two anchoring wires are coupled with the hollow cap of the implant and have different lengths corresponding to the structures of the prostatic urethra to which they are to anchor to. The first anchoring wire is long enough such that when the implant is positioned within the prostatic urethra, the first anchoring wire places pressure of the verumontanum, thereby preventing the implant from migrating towards the bladder. The first anchoring wire essentially holds the implant in place without having to permanently affix the implant to tissues within the prostatic urethra (for example, without having to use a glue, sutures or other means for coupling implants with tissues in the body). The second anchoring wire is long enough such that when the implant is positioned within the prostatic urethra, the second anchoring wire places pressure of the bulbous and dangling end of the median lobe, thereby preventing the bulbous and dangling end of the median lobe from being sucked towards the bladder and obstructing the bladder neck after urination. Given the anatomical structure of the prostate and the prostatic urethra and that the implant is inserted into the urethra from the penis, the second anchoring wire is longer than the first anchoring wire as the bulbous end of the median lobe is more distal to the penis than the verumontanum.

In a second embodiment of the disclosed technique, the implant has a single anchoring wire having two extensions, with the anchoring wire having a fork-like shape. A first extension is used to anchor the implant to the prostatic urethra, for example, by applying pressure to the verumontanum whereas a second extension is used to apply pressure to the bulbous end of the median lobe. This embodiment is similar to the embodiment described above however in this embodiment the anchoring wire has a monolithic structure and only one coupling point with the hollow cap. In this embodiment, each extension fulfills the function of the respective anchoring wire in the previous embodiment.

In a third embodiment of the disclosed technique, the implant has a single anchoring wire having a shape resembling the outline of a leaf, a leaflet or a teardrop, for applying pressure to the bulbous end of the median lobe. The single anchoring wire in this embodiment is similar to the second anchoring wire in the first embodiment mentioned above. In this embodiment, other means are used to maintain the position of the implant within the prostatic urethra and preventing it from migrating towards the bladder. For example, in this embodiment, a biological glue, sutures and/or pins can be used to anchor the implant within the prostatic urethra.

It is noted that according to the disclosed technique, since the implant has an anchoring wire or extension for preventing the bulbous end of the median lobe from moving, the implant of the disclosed technique is meant to remain implanted within the prostatic urethra either permanently or semi-permanently. This is because the implant of the disclosed technique does not reduce the size of and/or change the composition of an enlarged median lobe, therefore the implant must remain permanently in place to prevent the bulbous end of the median lobe from causing BNO. In the event that the median lobe reduces in size (for example, through medication, through surgery or through other techniques) and the bulbous end of the median lobe is no longer a worry for causing BNO, then the implant of the disclosed technique can be removed from the prostatic urethra. In this respect, the implant of the disclosed technique can be semi-permanent.

In accordance with another embodiment of the disclosed technique, there is thus provided a method for deploying a prostatic urethra implant in the prostatic urethra of a patient. The method involves enfolding the implant within a sheath. The implant is elastic and thereby conforms to the circumference of the enfolding sheath which is smaller than the circumference of the implant when expanded. The sheath is inserted into the urethra and is pushed until its distal end extends into the bladder of the subject. The implant is pushed within the sheath until it extends from the distal end of the sheath and is thus released from the sheath. Once released, the elastic implant resumes its original, extended configuration due to the shape memory material it is made from. The implant is then pulled into the prostatic urethra and positioned in its place. Positioning the implant can include in physical location within the prostatic urethra as well as its rotational orientation. As described below, the implant can be rotated within the prostatic urethra in order to properly place the anchoring wires in their respective positions for anchoring the implant to the verumontanum and for apply pressure to the bulbous end of the median lobe. Once the implant is properly positioned, the sheath is removed from the urethra.

As mentioned above, the implant can include a proximal cap which is hollow, having a niche (or a protrusion). The niche is non-round that can transfer rotary motion from a corresponding pin (or in case of a protrusion - a corresponding niche). Thus the implant can be rotated within the prostatic urethra to a desired rotary orientation.

As mentioned, the implant is pulled back from the bladder in the proximal direction until it is positioned within the prostatic urethra (and/or the bladder neck). The implant remains either permanently or semi-permanently within the prostatic urethra for a period of time. During this time, the incising wires in the implant create longitudinal incisions in the surrounding tissues of the inner wall of the prostatic urethra for relieving urethral constrictions whereas one of the anchoring wires applies pressure to the bulbous end of the median lobe. If the implant is to be removed from the patient, a sheath is inserted into the urethra and compresses the implant, thereby folding the implant back into its compressed configuration. Following this, the implant can be removed from the urethra via the sheath.

In this description, the terms pressure and force (e.g., applying radial pressure or applying radial force) are employed interchangeably herein below, to describe the operation of the wires of the implant on the surrounding tissues and anatomical landmarks in the prostatic urethra. That is, the wires (incising and anchoring) are described as applying pressure on the tissues, or as applying an outward radial force on the tissues. Herein below, the terms proximal and distal refer to directions relative to the implantable device and the delivery system. In particular, the distal end is the end of the device (or of the system) that is inserted into the body of the patient first and reaches the deepest. The proximal end is the end closer to the exit from the body of the patient. Thus in reference to the disclosed technique, the bladder is the most distal point whereas the opening of the urethra in the penis is the most proximal point.

It is noted as well that disclosed technique is in general described using the embodiment having two anchoring wires having the worker skilled in the art can easily understand how the description can be modified to apply the disclosed technique to the other embodiments mentioned above. This applies to the embodiment having a single anchoring wire with two extensions as well as the embodiment having a single anchoring wire and no anchoring wire for anchoring the implant within the prostatic urethra. In addition, the disclosed technique is described with reference to the human male reproductive system however the disclosed technique (i.e., the implant and its methods of delivery) can equally be applied to the reproductive systems of male animals having a prostate gland or a gland which is anatomically and homologously similar to the human male prostate.

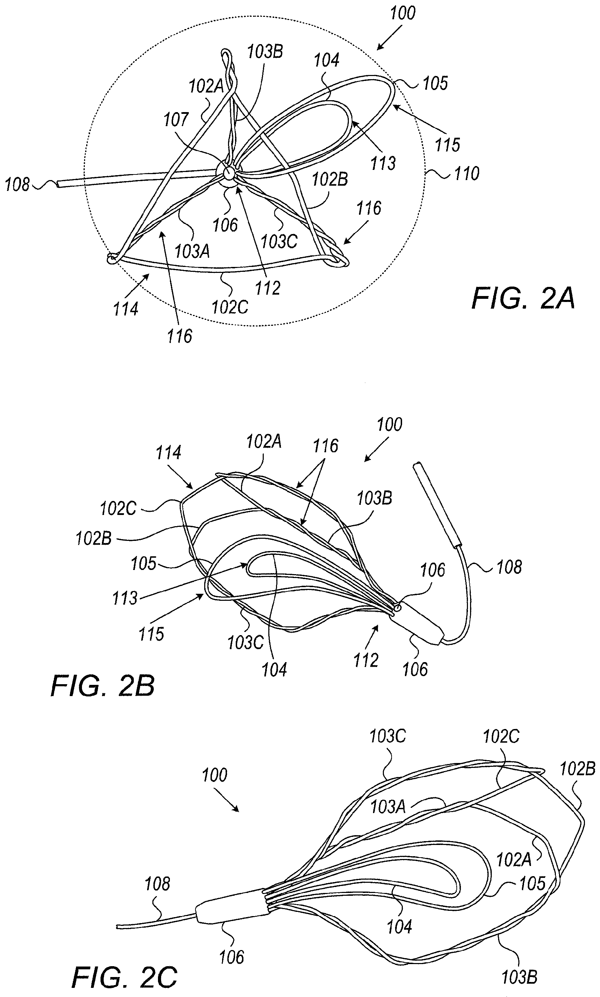

Reference is now made to Figures 2A, 2B and 2C, which are schematic illustrations of a prostatic urethra implant, generally referenced 100, for creating incisions in the tissues of the inner wall the prostatic urethra and for compressing the median lobe, constructed and operative in accordance with an embodiment of the disclosed technique. Figure 2A depicts the implant from a top view perspective (i.e., as would be seen in case the observer is located distally to the implant) and Figures 2B and 2C depict the implant from opposite isometric perspectives. Implant 100 includes three closed-shaped wires 102A, 102B and 102C, a first anchoring wire 104, a second anchoring wire 105, a proximal cap 106 and an extraction string 108.

The closed shape of each of wires 102A-102C can be broadly divided into a proximal section 112, a distal section 114 and two lateral sections 116 extending between the proximal section and the distal section. The proximal, distal and lateral sections are numbered for only one of wires 102A-102C to reduce clutter in Figures 2A-2B. As shown, proximal section 112 can be a Li-shaped proximal end, from which lateral sections 116 extend. Distal section 114 is the section connecting lateral sections 116. Each of wires 102A-102C is coupled with adjacent ones of wires 102A-102C on either side thereof. Thus as shown, the lateral sections of each of wires 102A-102C are coupled with lateral sections of adjacent wires, respectively shown as coupled lateral sections 103A, 103B and 103C. For example, one lateral section of wire 102A is coupled with a lateral section of wire 102B as coupled lateral section 103B, and the other lateral section of wire 102A is coupled with a lateral section of wire 102C as coupled lateral section 103A. The other lateral section of wire 102B (not coupled with wire 102A) is coupled with the other lateral section of wire 102C (that is also not coupled with wire 102A) as coupled lateral section 103C.

Proximal cap 106 holds the proximal ends of wires 102A-102C together. As shown, proximal cap 106 has a hollow 107. Since proximal cap 106 is placed within the urethra it must be hollow to enable urine to pass through the urethra. Extraction string 108 is coupled with either wires 102A-102C, with proximal cap 106 or with both. As shown as shown, first anchoring wire 104 and second anchoring wire 105 both have shapes resembling the outline of a leaf. First anchoring wire 104 and second anchoring wire 105 can likewise have shapes resembling the outlines of a leaflet, a teardrop or similar shapes for exerting an outward radial pressure. Similar to proximal cap 106, first anchoring wire 104 and second anchoring wire 105 have outline shapes which are substantially hollow, thus not impending the passage of any liquids and/or fluids through the prostatic urethra where they are positioned. First anchoring wire 104 acts as an implant anchor for anchoring implant 100 in the prostatic urethra. Second anchoring wire 105 acts as a median lobe compressor for applying pressure to the bulbous end of the median lobe and for preventing it from moving around, especially after urination.

The following paragraphs describe the use of implant 100. Thereafter, the components of implant 100 are described in more detail. Implant 100 is permanently, or semi-permanently implanted in the prostatic urethra for creating longitudinal incisions in the tissues of the inner wall of the prostatic urethra, thereby relieving urethra constriction and also for applying pressure to the bulbous end of the median lobe for preventing BNO.

Implant 100 is implanted by employing a sheath (not shown) for inserting the implant into the urethra. Implant 100 is compressed within the sheath such that the diameter of the circumference of implant 100 when expanded, illustrated by a dotted circle 110, conforms to the inner diameter of the sheath. Wires 102A-102C and anchoring wires 104 and 105 are made of elastic material having shape memory, such that they can be compressed and such that when released from the enfolding sheath they regain their original, extended shape and configuration, expanding to the original circumference diameter of dotted circle 110. When positioned in the prostatic urethra, the expanded diameter and configuration of implant 100 is bound by the inner diameter of the urethral walls surrounding it.

Wires 102A-102C push against the surrounding tissues (i.e., apply an outward radial force to the tissues of the prostatic urethra). Over time, the force applied by wires 102A-102C impairs the blood (and oxygen) supply to the portion of the tissues in direct contact with wires 102A-102C, thereby inducing tissue necrosis and creating infarcted incisions. Over time, the incisions become deeper until wires 102A-102C reach their full expansion (i.e., until implant 100 regains its original circumference diameter as illustrated by dotted circle 110). Once fully expanded, constriction of the prostatic urethra is substantially relieved. First anchoring wire 104 is positioned such that a distal end 113 of first anchoring wire 104 is distal to the verumontanum of the prostatic urethra. Distal end 113 substantially ensures that implant 100 does not move and/or migrate towards the bladder once implanted. Second anchoring wire 105 is positioned such that a distal end 115 of second anchoring wire 105 is distal to the bulbous end of the median lobe. Distal end 115 compresses the bulbous end of the median lobe and ensures that it does not move around, especially after urination when suction is generated in the prostatic urethra.

Implant 100 is implanted such that wires 102A-102C and anchoring wires 104 and 105 are aligned with the longitudinal direction of the urethra. Therefore, wires 102A-102C create longitudinal incisions in the tissues of the inner wall of the prostatic urethra, running along the urinary passage.

The period of time required for creating incisions that are sufficient to relieve urethra constriction depends on various factors, such as the level of constriction, the materials of wires 102A-102C, the size of the original fully extended shape of wires 102A-102C and the like. As mentioned above, implant 100 can remain in the prostatic urethra indefinitely or for a predetermined period of time (i.e., semi-permanently). The incisions created by implant 100 are created over time without causing pain or bleeding to the patient. After implant 100 is implanted, the patient can be released and resume his regular lifestyle, without any hindrances. The pressure applied by anchoring wires 104 and 105 is sufficient for maintaining the position of implant 100 in the prostatic urethra and for compressing the median lobe without causing any pain and/or discomfort to the patient. In addition, the shape of anchoring wires 104 and 105 is unobtrusive (as the shapes of the anchoring wires are substantially hollow), thereby enabling fluids and liquids (such as urine, prostatic secretions, semen and the like) to pass over and through anchoring wires 104 and 105 without hindrance.

Implant 100 is implanted within the prostatic urethra to relieve constriction of the urethra and for compressing an enlarged median lobe having a bulbous end, both caused for example by prostate enlargement. Implant 100 can be positioned in other, or in additional, areas of the urinal passage, such as in the bladder neck. Depending on the given desired placement, the length of anchoring wires 104 and 105 may need to be altered accordingly to serve their respective functions of anchoring and compressing. Alternatively, implant 100 can be implanted in any tubular organ that requires relief of a constriction while simultaneously restricting the mobility of anatomical structures, such as tubular organs of the digestion system, blood vessels and the like.

Wires 102A-102C as well as anchoring wires 104 and 105 are closed-shaped wires made of elastic material. The material of all the wires of the disclosed technique (incising and anchoring) should be elastic enough to allow the wires to be compressed within a sheath, and to conform to the inner diameter of the sheath during insertion into the urethra. The wires also require shape memory such that they regain their original, extended shape and configuration (and their original circumference diameter) once released from the sheath. Additionally, the incising wires should be strong enough to apply a force on the surrounding tissues to induce necrosis in the tissues (e.g., a force of 0.5 Newtons) and thereby to create infarcted longitudinal incisions. The anchoring wires should be strong enough to apply enough of a radial force to compress the median lobe and the verumontanum such that the passage of fluids and liquids through the urethra does not dislodge the anchoring wires. Wires 102A-102C and anchoring wires 104 and 105 can be made, for example, from Nickel Titanium alloy (Nitinol). All parts of implant 100 should be made from biocompatible materials, such that there is no danger of infection to body of the patient from implant 100.

As mentioned above, the closed shape of the incising wires can broadly be divided into three sections, proximal section 112, a middle section consisting of lateral sections 116, and distal section 114. The distal section serves as a support crosspiece connecting the lateral sections of the incising wires. Exemplary closed shapes of the incising wires are illustrated in Figures 2A-2C, 3A-3C and 4.

The lateral sections of each of wires 102A-102C (shown as coupled lateral sections 103A-103C) are the sections in contact with the surrounding tissues of the prostatic urethra. That is, the lateral sections are the sections pushing against the tissues for creating the incisions. The lateral sections of each of wires 102A-102C are coupled (i.e., adjoined, braided and/or attached) with lateral sections of adjacent wires as mentioned above. In this manner, the adjoined wires together form a supporting wire frame such that each closed-shape wire forms a face of the frame and each adjoined pair of lateral sections of adjacent wire_§ forms an edge of the frame.

When the lateral sections of wires 102A-102C are pushed against the surrounding tissues (i.e., as implant 100 tries to regain its original shape while being bound by the urethra inner walls of the prostatic urethra), the surrounding tissues apply an opposite force on wires 102A-102C in accordance with Newton's third law of motion. Each of wires 102A-102C is pushed against the adjacent wires to which it is adjoined. The wire frame increases the structural stability of implant 100, allowing implant 100 to apply sufficient force for creating the incisions in the surrounding tissues. Thus, the wire frame obviates the need for an additional support element, such as a central support tube.

In the example set forth in Figures 2A-2C, incising wires 102A-102C are adjoined together by being wound (i.e., twisted and/or braided) around each other. That is, the first lateral section of wire 102A and the first lateral section of wire 102B are wound around each other as coupled lateral section 103B, the first second lateral section of wire 102A and the first lateral section of wire 102C are wound around each other as coupled lateral section 103A and the second lateral section of wire 102B and the second lateral section of wire 102C are wound around each other as coupled lateral section 103C. The twist coupling of wires 102A-102C further provides structural solidity to implant 100. Thus, each of wires 102A-102C can be made thinner without compromising the robustness of implant 100. For example, each of wires 102A-102C can be as thin as 0.5 millimeters (i.e., the cross section of each of the wires is 0.5 millimeters).

The winding of wires 102A-102C can be achieved, for example, by twisting the lateral sections around each other and thermally treating implant 100 for stabilizing the windings. Wires 102A-102C can be wound around each other by being placed in a mold having rotating elements that grab the lateral sections and wind them around each other.

In the example set forth in Figures 2A-2C there are three wound wires, each consisting of two lateral sections of two adjacent wires, wound around each other. Thus, the wire frame has three lateral edges creating three longitudinal incisions. In accordance with an alternative embodiment of the disclosed technique, the implant can include other numbers of closed-shaped wires, such as a single wire, two wires (for a wire frame of two lateral edges creating two longitudinal incisions), four wires (for a wire frame of four lateral edges creating four longitudinal incisions), five wires and the like.

Proximal cap 106 is coupled with the proximal ends of wires 102A-102C for coupling wires 102A-102C together, thereby strengthening the wire frame. Put another way, proximal cap 106 helps to maintain the structure of implant 100 (i.e., increases the structural stability) by further adjoining wires 102A-102C to each other. As mentioned above, proximal cap 106 is also hollow and has an outer diameter which is similar to the diameter of an unconstricted urethra such that when positioned in the prostatic urethra, proximal cap 106 does not place any additional pressure on the inner walls of the urethra that are unnatural that may cause discomfort and/or pain to the patient.

In the example set forth in Figures 2A-2C, proximal cap 106 encases the proximal ends of wires 102A-102C. Thus, proximal cap 106 shields tissues of the urethra from getting caught in the proximal ends of wires 102A-102C. Additionally, proximal cap 106 serves to prevent wires 102A-102C from unwinding.

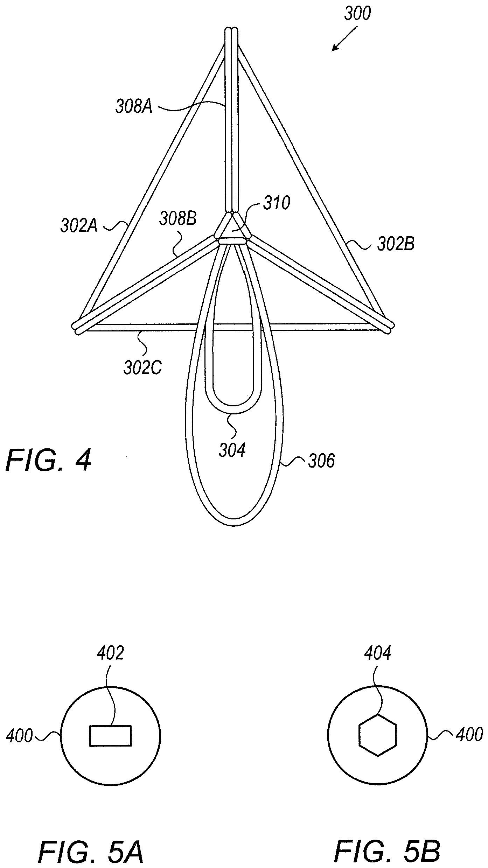

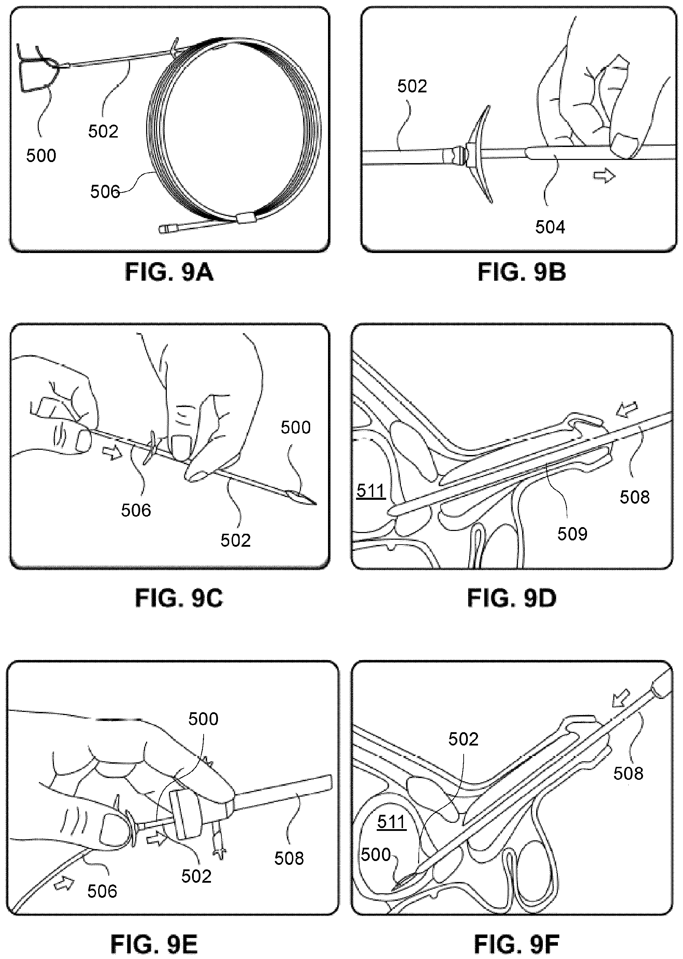

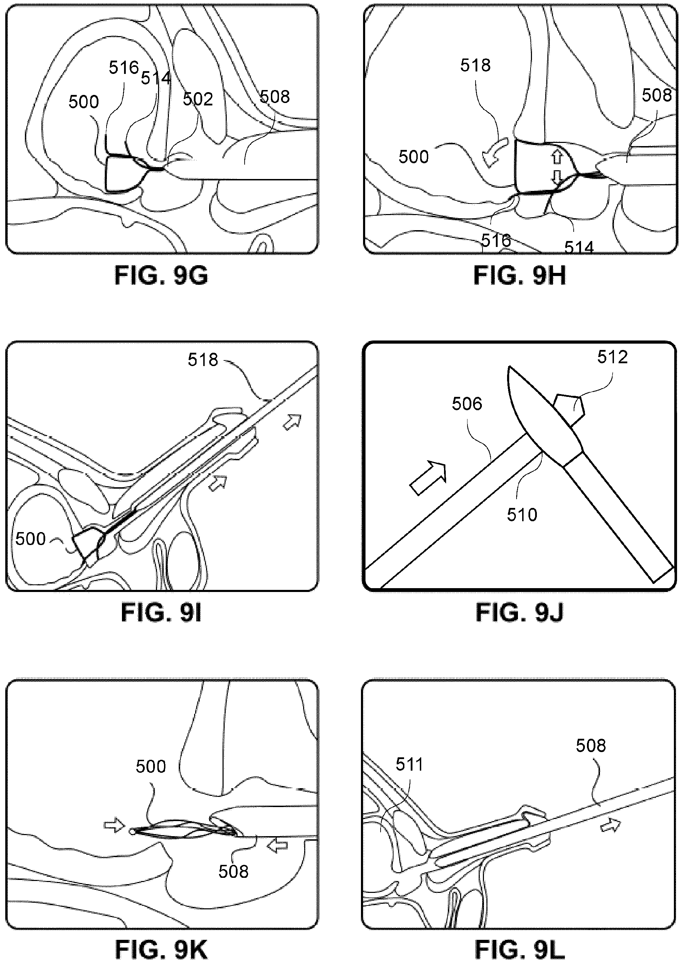

Proximal cap 106 includes hollow 107, which can also be described as a proximal non-round niche (e.g., niche 502 of Figures 5A and 5B). In general, hollow 107 does not have a round shape, thus enabling proximal cap 106 to rotate with the aid of a tool (not shown) having a complementary shape to hollow 107, and thereby enabling the orientation of incising wires 102A-102C and anchoring wires 104 and 105 to be changed. The non-round proximal niche of proximal cap 106 is configured to receive a corresponding non-round pin (i.e., a tool) and to transfer rotational motion of the pin to implant 100. Thereby, a physician or user can rotate implant 100 when implant is located within the bladder of the subject, as will be detailed further below with reference to Figures 5A-5B and 9A-9L.

As mentioned above first anchoring wire 104 serves as a one-way stopper allowing implant 100 to move from the bladder into the prostatic urethra yet preventing implant 100 from migrating back towards the bladder. This can be achieved by first anchoring wire 104 having a wide enough shape when fully expanded such that it will get stuck against one of the urethral sphincters. This can also be achieved by first anchoring wire 104 applying an outward radial pressure against the verumontanum of the prostatic urethra. First anchoring wire 104 can be a wire leaflet (e.g., as depicted in Figures 2A-2C), or any other form allowing it to move across the urethral sphincters in the proximal direction while preventing it from moving across the urethral sphincters in the distal direction. Said otherwise, first anchoring wire 104 should have a shape affording single directionality regarding its movement in the proximal direction (i.e., towards the opening of the urethra in the penis). First anchoring wire 104 can be coupled to implant 100 elastically or via an axis. First anchoring wire 104 can also be coupled via another coupling mechanism configured to enable first anchoring wire 104 to serve as a one-way stopper for movement across the urethral sphincters. Alternatively, another or additional anchoring elements can be employed for anchoring implant 100 in its place (preventing movement in the proximal direction, the distal direction or both), such as barbs (not shown) on wires 102A-102C. As mentioned above, in such an embodiment, implant 100 may only include second anchoring wire 105, with the first anchoring wire having been replaced by another mechanism for anchoring implant 100 within the prostatic urethra and for preventing it from migrating towards the bladder.

Second anchoring wire 105 serves to apply pressure to the bulbous end of the median lobe, thereby preventing the bulbous end from moving towards the bladder neck after urination.

Extraction string 108 enables the physician to extract implant 100. Specifically, the distal end of string 108 is coupled with implant 100 and the proximal end of string 108 extends (slightly) outside of the body of the patient. The physician can insert an extraction sheath into the urethra along string 108 for enfolding and compressing implant 100. The physician can extract the enfolded implant by pulling on string 108. String 108 is strong enough for pulling implant 100 without being torn (e.g., the thickness and materials of string 108 allow pulling implant 100 via string 108). String 108 can be a single strand or a woven bundle of strands for further fortifying it. As described below, the extraction sheath is inserted into the urethra over string 108 and guided to implant 100. By pulling on string 108, implant 100 will then be pulled into the extraction sheath and compressed into a shape that fits into the extraction sheath, as it was when implant 100 was initially deployed. Removal of the extraction sheath with implant 100 compressed therein is then possible by pulling on string 108 and the extraction sheath.

Implant 100 is deployed such that it does not extend distally beyond the bladder neck of the subject (i.e., does not extend into the bladder). Specifically, wires 102A-102C do not come into contact with the tissues of the bladder itself. Thereby, implant 100 does not irritate the bladder of the patient.

In accordance with an embodiment of the disclosed technique, the wires of implant 100 can be coloured in such a manner that enables the physician to easily position it when deployed in the prostatic urethra. For example, the incising wires of the implant are colour-coded such that sections that should be positioned on the superior side of the prostatic urethra are coloured blue and sections that should be positioned on the inferior side of the prostatic urethra are colored white. The physician can observe the implant in the bladder via a cystoscope and rotate the implant to the desired orientation according to the colours of the implant. Likewise, anchoring wires 104 and 105 can be colour-coded so that they are properly positioned respectively distal to the verumontanum and distal to the bulbous end of the median lobe. As just mentioned, by using a cystoscope, the physician can observe and verify the position of the anchoring wires as well to ensure proper placement.

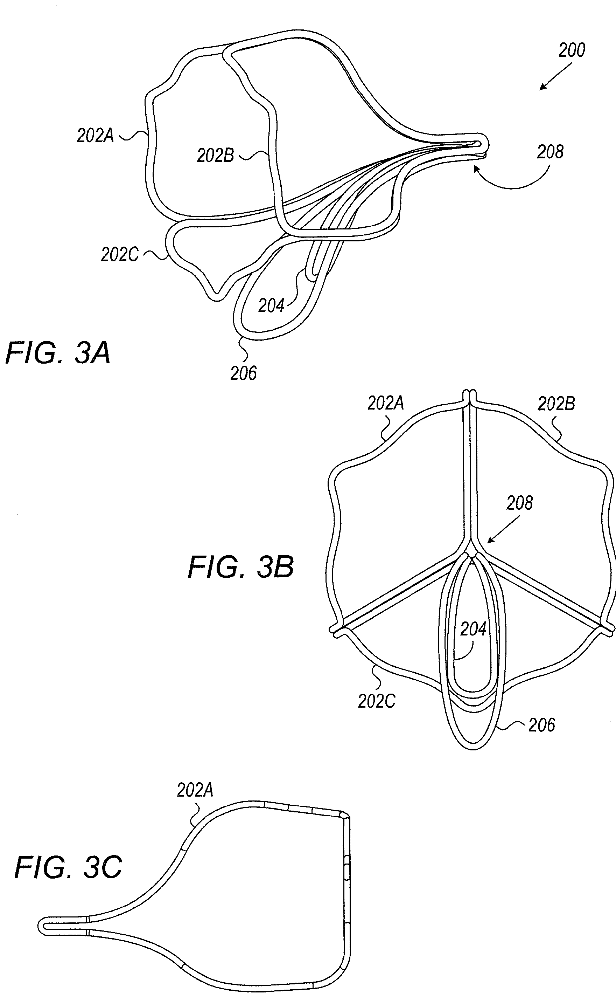

Reference is now made to Figures 3A, 3B and 3C, which are schematic illustrations of a second prostatic urethra implant, generally referenced 200, for creating incisions in the tissues of the inner wall of the prostatic urethra, constructed and operative in accordance with another embodiment of the disclosed technique. Figure 3A depicts the implant from an isometric perspective, Figure 3B depicts the implant from a top-view perspective and Figure 3C depicts one of the closed-shaped wires of the implant. Second implant 200 is substantially similar to implant 100 (Figures 2A-2C), except that second implant 200 does not include a proximal cap and the lateral sections of the incising wires are adjoined together without being wound together, as described below. Implant 200 includes three closed-shaped incising wires 202A, 202B and 202C, a first anchoring wire 204 and a second anchoring wire 206. The proximal end of implant 200 is indicated by an arrow 208. The components of implant 200 are similar to those of implant 100 and thus for the sake of brevity only the differences are elaborated herein below.

The closed shape of each of wires 202A-202C is depicted in Figure 3C. The closed shape is truncated at the distal end thereof. Thus the distal end of each of wires 202A-202C is substantially perpendicular to the longitudinal axis of implant 200. Therefore the incising wires do not come into contact with the tissues of the bladder and thus prevent bladder irritation.

As shown, wires 202A-202C are not wound around each other. Instead, wires 202A-202C can be adjoined to one another (i.e., the lateral sections are adjoined to lateral sections of adjacent wires) by various manners. For example, the incising wires can be welded together, glued together or coupled by a coupling mechanism or element (e.g., a coupling thread binding the lateral sections together).

In the example set forth in Figures 3A-3C (and in Figure 4 herein below), the implant is shown without a proximal cap and an extraction string. It is noted however, that the implant can include any of the proximal cap, the extraction string or both. Noted as well is the relative positioning of the first and second anchoring wires. As shown, first anchoring wire 204 is shorter than second anchoring wire 206. The proximal ends of incising wires 202A-202C and first and second anchoring wires 204 and 206 are all coupled together at proximal end 208 of implant 200. The wires (202A-202C, 204 and 206) can be coupled via welding, gluing, a coupling thread and the like.

Reference is now made to Figure 4, which is a schematic illustration of a third prostatic urethra implant, generally referenced 300, for creating incisions in the tissues of the inner wall of the prostatic urethra, constructed and operative in accordance with a further embodiment of the disclosed technique. Implant 300 includes three closed-shaped incising wires 302A, 302B and 302C, a first anchoring wire 304 and a second anchoring wire 306. The components of implant 300 are similar to those of implant 200 (Figures 3A-3C) and for the sake of brevity only the differences are elaborated upon herein below. Implant 300 is depicted from a bottom-view perspective (i.e., as seen by a proximally located observer). The closed shape of wires 302A-302C is triangular, such that together wires 302A-302C form a triangular pyramidal wire frame with the proximal ends of the wires forming the apex of the pyramid, shown by an arrow 310, and the distal ends forming the base of the pyramid. As shown and numbered for incising wire 302A, its lateral sections 308A and 308B are adjoined to neighboring lateral sections (not labeled) of incising wires 302B and 302C. The adjoined lateral sections of wires 302A-302C form the lateral edges of the triangular pyramid. As can be understood by the worker skilled in the art, other wire frame shapes are possible for the prostatic urethra implant of the disclosed technique, such as a square-based pyramid, a pentagonal-based pyramid, a polygonal-based pyramid and the like.

Reference is now made to Figures 5A and 5B, which are schematic illustrations of a proximal niche, generally referenced 402 and 404 respectively, of a proximal cap of a prostatic urethra implant, constructed and operative in accordance with yet another embodiment of the disclosed technique. Figures 5A and 5B show a proximal cap 400, which is substantially similar to proximal cap 106 Figures 2A-2C). As shown, the proximal niche has a hollow non-round shape for allowing it to transfer rotational motion to the implant from a corresponding pin or tool (not shown) inserted into the niche. The hollow shape enables liquids and fluids to flow through the proximal cap. Thus the physician can rotate the implant from a position outside the patient even when the implant is already inserted into the urethra (e.g., when the implant is in the bladder). In the example set forth in Figure SA, the shape of niche 402 is rectangular and in the example set forth in Figure 5B, the shape of niche 404 is hexagonal. Alternatively, the proximal niche can have any shape allowing it to transfer rotational motion (i.e., rotation around the central axis (not labeled) of the proximal cap), such as non-round shapes, a slit, an array of niches (e.g., two holes) and the like.

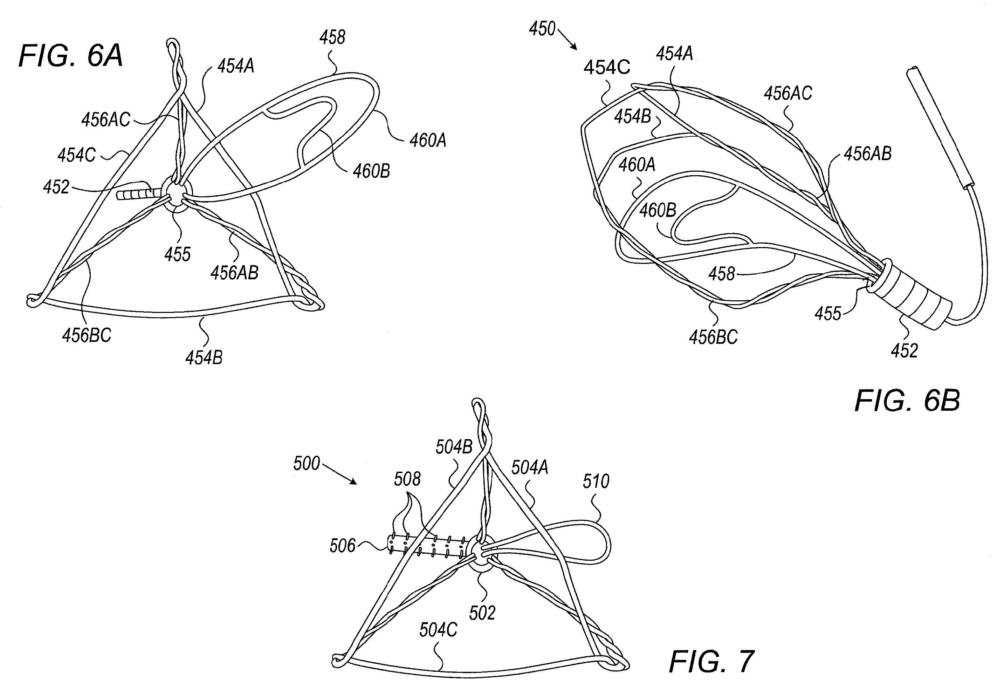

Reference is now made to Figures 6A and 6B, which are schematic illustrations of a fourth prostatic urethra implant, generally referenced 450, for creating incisions in the tissues of the inner wall of the prostatic urethra, constructed and operative in accordance with yet a further embodiment of the disclosed technique. Figure 6A depicts the implant from a top view perspective (i.e., as would be seen in case the observer is located distally to the implant) and Figure 6B depicts the implant from an isometric perspective. Implant 450 includes three closed-shaped incising wires 454A, 454B and 454C, a double pronged anchoring wire 458 and a proximal cap 452. Implant 450 is substantially similar to implants 100 (Figures 2A-2C), 200 (Figures 3A-3C) and 300 (Figure 4). Proximal cap 452 includes a hollow 455 and as shown, the lateral sections of each two adjacent incising wire are coupled together, shown as laterals sections 456AB (coupling the lateral sections of wires 454A and 454B), 456BC (coupling the lateral sections of wires 454B and 454C) and 456AC (coupling the lateral sections of wires 454A and 454C). For the sake of brevity, only the differences between implant 450 and implants 100, 200 and 300 will be elaborated upon.

As can be seen, implant 450 includes a single anchoring wire, labeled as double pronged anchoring wire 458, which includes a first extension 460A and a second extension 460B. Double pronged anchoring wire 458 is functionally equivalent to first anchoring wire 104 (Figures 2A-2C) and second anchoring wire 105 (Figures 2A-2C), with first extension 460A being functionally similar to second anchoring wire 105 and second extension 460B being functionally similar to first anchoring wire 104. Thus first extension 460A functions as a median lobe compressor whereas second extension 460B functions as an anchor with the verumontanum for preventing implant 450 from migrating towards the bladder once implanted. Said otherwise, double pronged anchoring wire 458 is a monolithic anchoring wire having two extensions, one for anchoring the implant to the verumontanum and the other for compressing the median lobe. Similar to first and second anchoring wires 104 and 105, double pronged anchoring wire 458 can be made from an elastic material having shape memory, such as Nitinol. Thus the main difference in this embodiment is that the anchoring wire in a single wire attached to proximal cap 452 instead of two separate anchoring wires attached to the proximal cap, as shown for example in Figures 2A-2C. The form of double pronged anchoring wire 458 (i.e., as a single anchoring wire) can be used in the wire frame shapes and configurations of implants 200 (Figures 3A-3C) and 300 (Figure 4) as well.

Reference is now made to Figure 7, which is a schematic illustration of a fifth prostatic urethra implant, generally referenced 500, for creating incisions in the tissues of the inner wall of the prostatic urethra, constructed and operative in accordance with yet another embodiment of the disclosed technique. Figure 7 depicts the implant from a top view perspective (i.e., as would be seen in case the observer is located distally to the implant). Implant 500 includes three closed-shaped incising wires 504A, 504B and 504C, an anchoring wire 510 and a proximal cap 502. Implant 500 is substantially similar to implants 100 (Figures 2A-2C), 200 (Figures 3A-3C), 300 (Figure 4) and 450 (Figures 6A-68). A body 506 of proximal cap 502 is shown. Similar to the other implants of the disclosed technique, the lateral sections of each two adjacent incising wire are coupled together (not labeled). For the sake of brevity, only the differences between implant 500 and implants 100, 200, 300 and 450 will be elaborated upon.

As can be seen, implant 500 includes only a single anchoring wire, which is functionally similar to second anchoring wire 105 (Figures 2A-2C), thereby serving the function of compressing the median lobe. Implant 500 thus does not include an anchoring wire for preventing its migration towards the bladder. Instead of an anchoring wire for that purpose, body 506 includes a plurality of barbs 508 (as an example) for keeping proximal cap 502 within the prostatic urethra. Plurality of barbs 508 will prevent implant 500 from migrating towards the bladder. When a sheath is placed over implant 500, plurality of barbs 508 are covered and not in contact with the tissues of the inner wall of the urethra. Thus implant 500 can be inserted and retracted from the urethra using a sheath (not shown) which covers plurality of barbs 508 and prevents the abrasion of plurality of barbs 508 against the tissues of the inner wall of the urethra. As mentioned above, other mechanisms (instead of plurality of barbs 508) can be used to secure the position of implant 500 such that it does not migrate towards the bladder once implanted.

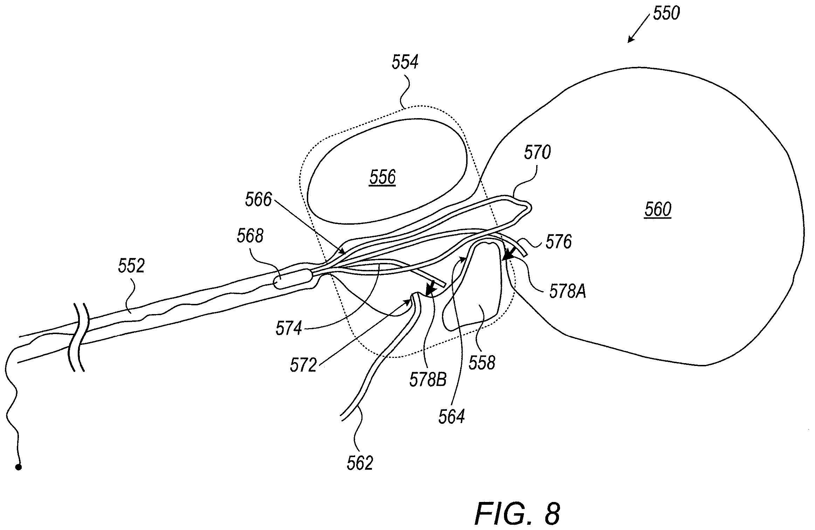

Reference is now made to Figure 8, which is a schematic illustration showing the placement of a prostatic urethra implant within the prostatic urethra, generally referenced 550, constructed and operative in accordance with yet a further embodiment of the disclosed technique. Figure 8 shows a sagittal plane cross-section of the prostatic urethra with an implant 566 (substantially similar for example with implant 100 of Figure 2A) of the disclosed technique positioned therewithin. A urethra 552 is shown, which enters a prostate 554, having a posterior lobe (not shown), a lateral lobe 556, a median lobe 558 and an anterior lobe (not shown). An ejaculatory duct 562 enters prostate 554 between the posterior lobe and median lobe 558. Median lobe 558 has enlarged thereby presenting a bulbous and dangling end 564. A verumontanum 572 of the prostatic urethra is also shown as well as a bladder 560.

Implant 566 includes a plurality of incising wires, shown schematically as an incising wire 570, a first anchoring wire 574, a second anchoring wire 576 and a proximal cap 568. Once implanted, as shown, proximal cap 576 sits proximally in the prostatic urethra at the opposite end of the bladder neck (not shown). Incising wire 570 sits within the prostatic urethra and applies an outward radial force along the tissues of the inner wall of the prostatic urethra. As mentioned above, this force causes incisions in the inner wall of the prostatic urethra, leading to infarction and relieving constricting pressure from lateral lobe 556 of the prostate. First anchoring wire 574 is long enough to exert a pressure on verumontanum 572, exerting an outward radial force indicated by an arrow 5788 whereas second anchoring wire 576 is even longer than first anchoring wire 574 and exerts an outward radial force on bulbous end 564, indicated by an arrow 578A. Radial force 5788 is sufficient to keep implant 566 from migrating towards the bladder neck and radial force 578A is sufficient to keep bulbous end 564 from moving around, especially after urination wherein it can be sucked towards the bladder neck and can cause 8NO. Proximal cap 568 is hollow thus enabling fluids and liquids to pass there through. As mentioned above, implant 566 is either permanent or semi-permanent and remains within the prostatic urethra.

Reference is now made to Figures 9A-9L, which are schematic illustrations of a method for deploying and for extracting a prostatic urethra implant, operative in accordance with yet another embodiment of the disclosed technique. With reference to Figure 5A, shown is a prostatic urethra implant 500 of the disclosed technique, along with a deployment sheath 502 and a guidewire 506. Implant 500 includes a proximal cap having a non-round proximal niche (both not shown). Guidewire 506 includes a distal head (i.e., distal pin) whose shape corresponds to the shape of the non-round proximal niche of the proximal cap of implant 500. Guidewire 506 also includes an inner channel (not show). The distal head of guidewire 506 is inserted into the non-round proximal niche of the proximal cap of implant 500. Implant 500 is attached to the distal end of deployment sheath 502 such that guidewire 506 runs through deployment sheath 502.

With reference to Figure 5B, a physician removes a protective cover 504 from implant 500 thereby causing implant 500 to expand to its expanded and open configuration (as seen in any of Figures 2A-2C, 3A-3C, 4, 6A-6B and 7). Protective cover 504 keeps implant 500 sterile during storage prior to use. With reference to Figure 5C, while holding guidewire 506, the physician pushes deployment sheath 502 over implant 500 thereby enfolding implant 500 (and thus compressing it) within deployment sheath 502 for delivery into the urethra.

With reference to Figure 5D, the physician inserts a rigid cystoscope 508 (e.g., size 20 French gauge) into the urethra 509, for example, as in a routine urethral catheterization procedure, until its distal end enters the bladder 511. With reference to Figure 5E, the physician inserts deployment sheath 502, including compressed implant 500 positioned inside the deployment sheath, into cystoscope 508. The physician continues pushing implant 500 through cystoscope 508 by pushing on guidewire 506 until implant 500 extends through the distal end of cystoscope 508. With reference to Figure 5F, the physician removes sheath 502 from implant 500 and out of cystoscope 508. This action substantially releases implant 500 such that it can expand to its open configuration. With reference to Figure 5G, once released from deployment sheath 502 and from cystoscope 508, implant 500 expands (i.e., regains its original extended shape). Shown in Figure 5G are a first anchoring wire 514 and a second anchoring wire 516.

With reference to Figure 5H, the physician rotates implant 500 to the desired orientation by rotating guidewire 506 (with its distal head being inserted into the proximal niche of implant 500), as shown by an arrow 518. First anchoring wire 514 and second anchoring wire 516 of implant 500 (e.g., first anchoring wire 104 and second anchoring wire 105 of Figures 2A-2C) should be positioned accordingly to be over the verumontanum and the bulbous end of the median lobe (both not labeled). The wires of implant 500 (incising wires and/or anchoring wires) can be color-coded such that the sections that should be positioned superiorly are colored, for example, blue and the sections that should be positioned inferiorly are colored, for example, white. The physician rotates implant 500 as detailed above using the proximal cap.

With reference to Figure 5I, while holding implant 500 in place by using guidewire 506, the physician retracts cystoscope 508. Thereafter, the physician pulls implant 500 via guidewire 506 until the first and second anchoring wires of implant 500 slide over the internal urethral sphincter. The physician continues to pull implant 500 via guidewire 506 into the prostatic urethra until the first anchoring wire is positioned over the verumontanum and the second anchoring wire is positioned over the bulbous end of the median lobe. Implant 500 is now positioned accordingly within the prostatic urethra. With reference to Figure 5J, in the case that implant 500 includes an extraction string 510, the physician cuts a knot 512 at the proximal end of extraction string 510, and retracts guidewire 506 from the urethra.

Thereby, implant 500 is implanted within the prostatic urethra and starts applying radial outward force on the surrounding tissues of the inner walls of the prostatic urethra for creating longitudinal incisions. In addition, the second anchoring wire compresses the bulbous end of the median lobe and holds it in place. Implant 500 is left permanently within the prostatic urethra or can be left semi-permanently. If needed, implant 500 can be removed as detailed below.

With reference to Figure 5K, for removing implant 500, the physician inserts cystoscope 508 through the urethra in the direction of bladder 511 towards implant 500 over extraction string 510. Alternatively, the physician can insert deployment sheath 502 instead of cystoscope 508 into the urethra. The physician pushes cystoscope 508 (and/or deployment sheath 502) until it enfolds and compresses implant 500. The implant is then positioned within cystoscope 508 and/or deployment sheath 502. With reference to Figure SL, the physician can then extract implant 500 enfolded within cystoscope 508 by pulling the implant via extraction string 510. Alternatively, the physician can insert a tool (not shown) within deployment sheath 502 for pulling and removing implant 500. Then, the physician extracts cystoscope 508 and/or deployment sheath 502 from the urethra.

It will be appreciated by persons skilled in the art that the disclosed technique is not limited to what has been particularly shown and described hereinabove. Rather the scope of the disclosed technique is defined only by the claims, which follow.

## Claims

1. Prostatic urethra implant for generating incisions in the prostatic urethra of a patient and for compressing the median lobe of the prostate, said implant comprising:
at least two closed-shaped incising wires, each of said incising wires having a proximal section, a distal section and two lateral sections extending longitudinally between said proximal section and said distal section, each of said lateral sections of each of said incising wires being adjoined with another lateral section of another one of said incising wires; and
a first closed-shaped anchoring wire, said first anchoring wire extending outwardly from at least one of said proximal sections and through one of said incising wires, said first anchoring wire being sufficiently long to compress said median lobe when said implant is positioned in said prostatic urethra,
wherein each of said at least two incising wires and said first anchoring wire being elastic and having shape memory thereby having an expanded configuration and also being compressible into a compressed configuration; and
wherein in said expanded configuration, said incising wires incising tissue in said prostatic urethra via an outward radial pressure and said first anchoring wire applying outward radial pressure on said median lobe.

2. The prostatic urethra implant according to claim 1, wherein each of said lateral sections of each of said incising wires are wound around said another lateral section of said another one of said incising wires.

3. The prostatic urethra implant according to claim 1, wherein each of said lateral sections of each of said incising wires are adjoined together via a coupling technique.

4. The prostatic urethra implant according to claim 3, wherein said coupling technique is selected from the list consisting of:
gluing;
welding; and
using a coupling thread for binding said lateral sections together.

5. The prostatic urethra implant according to claim 1, further comprising a hollow proximal cap coupled with said proximal section of each of said incising wires and with said first anchoring wire, said hollow proximal cap configured to hold said incising wires and said first anchoring wire together.

6. The prostatic urethra implant according to claim 5, wherein said hollow proximal cap comprises a proximal non-round niche configured to accept a corresponding mechanism; and wherein said proximal non-round niche is configured to transfer rotational motion of said corresponding mechanism to said implant.

7. The prostatic urethra implant according to claim 6, wherein said corresponding mechanism is selected from the list consisting of:
a pin; and
a tool.

8. The prostatic urethra implant according to claim 1, further comprising an extraction string coupled with said implant, said extraction string being arranged to allow pulling said implant out of said patient.

9. The prostatic urethra implant according to claim 1, wherein said at least two closed-shaped incising wires and said first anchoring wire are made from a material selected from the list consisting of:
Nickel Titanium alloy (Nitinol); and
a biocompatible material.

10. The prostatic urethra implant according to claim 1, further comprising a second anchoring wire, said second anchoring wire being shorter than said first anchoring wire, said second anchoring wire for preventing said implant, once implanted, from moving distally in the direction of the bladder neck of said patient.

11. The prostatic urethra implant according to claim 1, wherein said first anchoring wire comprises at least two extensions, a first one of said at least two extensions for compressing said median lobe and a second one of said at least two extensions for preventing said implant, once implanted, from moving distally in the direction of the bladder neck of said patient; and
wherein said first one of said at least two extensions is longer than said second one of said at least two extensions.

12. The prostatic urethra implant according to claim 5, said hollow proximal cap further comprising a plurality of barbs.

13. The prostatic urethra implant according to claim 1, wherein said at least two closed-shaped incising wires diverge at their respective distal sections such that said distal sections form a simple closed curve shape at a terminus of said implant in said expanded configuration.

14. The prostatic urethra implant according to claim 13, wherein said simple closed curve shape is selected from the list consisting of:
a triangle;
a square;
a pentagon; and
a polygon.
